**Europäisches Patentamt**

**European Patent Office**

**Office Européen des brevets**

(11) Veröffentlichungsnummer: **0 268 115 B1**

## (12) EUROPÄISCHE PATENTSCHRIFT

(45) Veröffentlichungstag der Patentschrift:
02.01.91 Patentblatt 91/01

(51) Int. Cl.⁵: **A61F 2/44**, A61F 2/28

(21) Anmeldenummer: **87115838.2**

(22) Anmeldetag: **28.10.87**

(54) Platzhalter, insbesondere für einen Wirbel.

(30) Priorität: 03.11.86 DE 3637314

(43) Veröffentlichungstag der Anmeldung:
25.05.88 Patentblatt 88/21

(45) Bekanntmachung des Hinweises auf die
Patenterteilung:
02.01.91 Patentblatt 91/01

(84) Benannte Vertragsstaaten:
AT BE CH DE ES FR GB GR IT LI NL SE

(56) Entgegenhaltungen:
DD-A- 245 863
DE-B- 2 404 214
DE-C- 3 023 942
US-A- 4 501 269

(73) Patentinhaber: **Biedermann, Lutz**
**Am Schäfersteig 8**
**D-7730 VS-Villingen (DE)**
Patentinhaber: **Harms, Jürgen, Prof. Dr.**
**Am Rüppurrer Schloss 5**
**D-7500 Karlsruhe (DE)**

(72) Erfinder: **Biedermann, Lutz**
**Am Schäfersteig 8**
**D-7730 VS-Villingen (DE)**
Erfinder: **Harms, Jürgen, Prof. Dr.**
**Am Rüppurrer Schloss 5**
**D-7500 Karlsruhe (DE)**

(74) Vertreter: **Prüfer, Lutz H., Dipl.-Phys.**
**Harthauser Strasse 25d**
**D-8000 München 90 (DE)**

## Beschreibung

Die Erfindung betrifft einen Platzhalter, insbesondere für einen Wirbel.

In einem Fall, in dem ein Wirbel aus der Wirbelsäule oder ein Knochenstück aus einem Knochen entfernt werden muß, ist es erforderlich, einen Platzhalter zwischen die verbleibenden Teile der Wirbelsäule bzw. des Knochens einzusetzen.

Aus der DE-C-30 23 942 ist ein als Implantat bezeichneter Platzhalter zum Einsetzen zwischen Wirbelkörpern der Wirbelsäule als Wirbelkörperersatz bekannt. Die beiden gegenüberliegenden Stützenden des Platzhalters sind tellerförmig ausgebildet und weisen zylinderförmige Vorsprünge auf. Die angrenzenden Wirbelkörper sind so ausgebildet, daß ein Verbinden der Stützenden mit den angrenzenden Wirbelkörpern möglich ist.

Aus der DE-A-34 35 771 ist eine Knochenmatrix bekannt, die zur Verbesserung der Induktion der Knochenbildung und der Stimulation der Knochenregeneration eine Vielzahl von Löchern aufweist. Die Matrix ist in Abhängigkeit von dem zu ersetzenden Stück plattenförmig, halbzylinderförmig oder zylinderförmig ausgebildet. Würde man eine derartige Knochenmatrix als Platzhalter zwischen zwei Wirbel setzen, müßte eine gesonderte Verbindung zu den benachbarten Wirbelkörpern geschaffen werden.

Aufgabe der Erfindung ist es, einen Platzhalter zu schaffen, der so ausgebildet ist, daß er sehr einfach zwischen zwei benachbarte Wirbel bzw. zwei benachbarte Knochenteile einsetzbar ist.

Diese Aufgabe wird durch einen Platzhalter gelöst, der ein mantelförmiges Element mit wenigstens einer Ausnehmung in der Wandung aufweist, wobei der obere und untere Rand des mantelförmigen Elementes jeweils wenigstens teilweise zackenförmig ausgebildet ist. Dadurch wird erreicht, daß eine drehstabile Verbindung zwischen den zu verbindenden Elementen herstellbar ist. Durch die Öffnung kann gewünschtenfalls ein in das Innere einzubringendes Material eingefüllt werden. Die Öffnung dient gegebenenfalls aber auch zur Aufnahme einer Pedikelschraube.

Weiterbildungen der Erfindung sind in den Unteransprüchen gekennzeichnet.

Weitere Merkmale und Zweckmäßigkeiten der Erfindung ergeben sich aus der Beschreibung von Ausführungsbeispielen anhand der Figuren. Von den Figuren zeigen :

Fig. 1 eine perspektivische Ansicht einer Ausführungsform der Erfindung ;

Fig. 2 eine Teilansicht der in Fig. 1 gezeigten Vorrichtung von oben ;

Fig. 3 eine der Fig. 2 entsprechende Darstellung einer abgewandelten Ausführungsform ;

Fig. 4 eine der Fig. 1 entsprechende Darstellung einer abgewandelten Ausführungsform ;

Fig. 5 eine der Fig. 2 entsprechende Darstellung der abgewandelten Ausführungsform ;

Fig. 6 eine Draufsicht von unten auf eine abgewandelte Ausführungsform ;

Fig. 7 eine Draufsicht von unten auf eine weitere Ausführungsform ;

Fig. 8 eine Draufsicht von unten auf eine Ausführungsform wie in Fig. 4 und 5, jedoch mit Bodenplatte ; und

Fig. 9 eine Seitenansicht des unteren Abschnittes der in Fig. 8 gezeigten Ausführungsform.

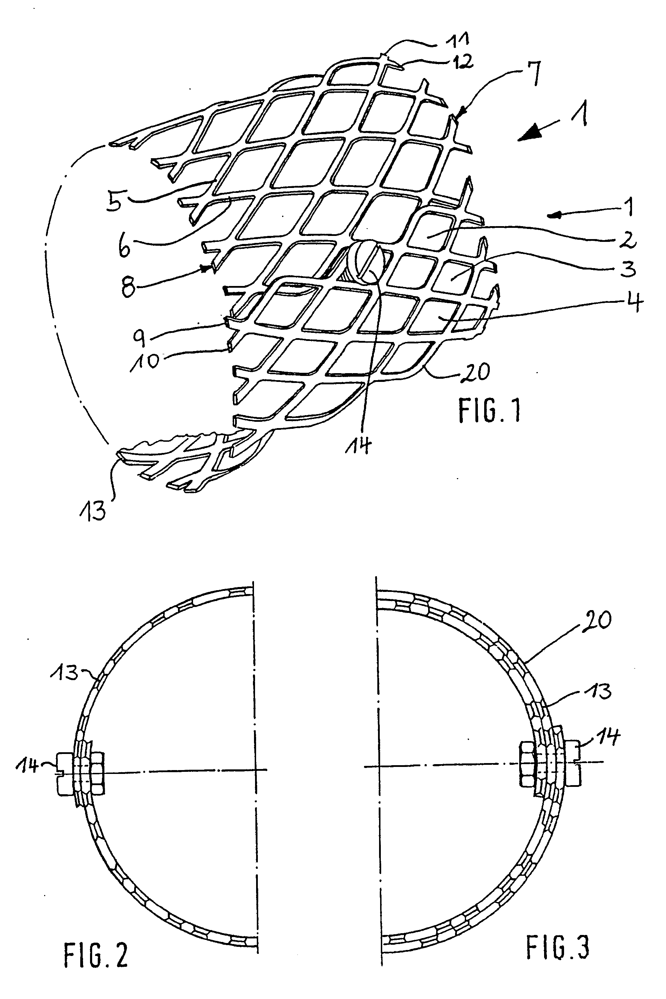

Wie insbesondere aus Fig. 1 ersichtlich ist, ist der Platzhalter 1 aus einem zylindermantelförmigen Element gebildet. Der Zylindermantel weist in der aus Fig. 1 ersichtlichen Weise sich mit ihrer Längsdiagonale parallel zur Zylinderachse erstreckende rautenförmige Ausnehmungen 2 auf. Jeweils benachbarte Reihen 3, 4 solcher Rauten sind gegeneinander um eine halbe Rautenhöhe versetzt. Dadurch wird ein Netz von sich unter einem spitzen Winkel schneidenden Bandstreifen 5, 6 gebildet, die unter jeweils gleich großen Winkeln gegen die Längsdiagonale der Rauten geneigt sind. Dadurch wird erreicht, daß eine auf den Platzhalter in Richtung seiner Längsachse wirkende Belastung gleichmäßig aufgenommen wird.

Der obere Rand 7 und der untere Rand 8 ist jeweils so ausgebildet, daß annähernd "V"-förmige Zacken 9, 10 bzw. 11,12 in der Mantelebene jeweils parallel zur Zylinderachse nach unten bzw. oben hervorstehen. Die Enden 13 der Zacken sind so angephast bzw. angeschrägt, daß sich die beiden schrägen Flächen unter einem Winkel von annähernd 45° schneiden, so daß eine Art Schneidrand gebildet ist. In dem in Figuren 1 und 2 gezeigten Ausführungsbeispiel ist der Mantel durch Zusammenrollen eines entsprechend ausgebildeten Blechstreifens gebildet. Die beiden sich überlappenden Enden sind durch eine Schraube 14 miteinander verbunden.

Grundsätzlich ist es möglich, gegebenenfalls die Wandung des Mantels zu verstärken, indem in der in Fig. 3 gezeigten Weise eine doppelte Wicklung verwendet wird.

Im Betrieb wird der Platzhalter in axialer Richtung anstelle eines herausgenommenen Wirbels oder Knochenstückes zwischen die verbleibenden Wirbel bzw. Knochenstücke eingesetzt. Dabei sind der Durchmesser und die axiale Länge des Mantels so gewählt, daß dieser so zwischen die verbleibenden Teile paßt, daß diese den ursprünglichen Abstand zueinander behalten. Der Platzhalter greift mit seinen Zacken 9, 10 bzw. 11, 12 in die Oberfläche der benachbarten Wirbel bzw. Knochen in der Weise ein, daß eine Torsionsbewegung des benachbarten einen Teiles gegenüber dem benachbarten anderen Teil durch die hervorstehenden Zacken auf das jeweils andere Teil übertragen bzw. gebremst wird.

Durch die Ausnehmungen hindurch wird zumindest das Innere des Elementes mit Knochenzement ausgefüllt. Gewünschtenfalls kann so viel Knochenzement eingeführt werden, daß dieser durch die Öffnungen nach außen tritt und daß eine Modellierung auf der Außenfläche vorgenommen wird. Vorzugsweise wird das Ausfüllen mit Knochenzement nach dem Einsetzen des oben beschriebenen Elementes vorgenommen. Es ist aber ein Ausfüllen des Innenraumes des Elementes vor dem Einsetzen möglich.

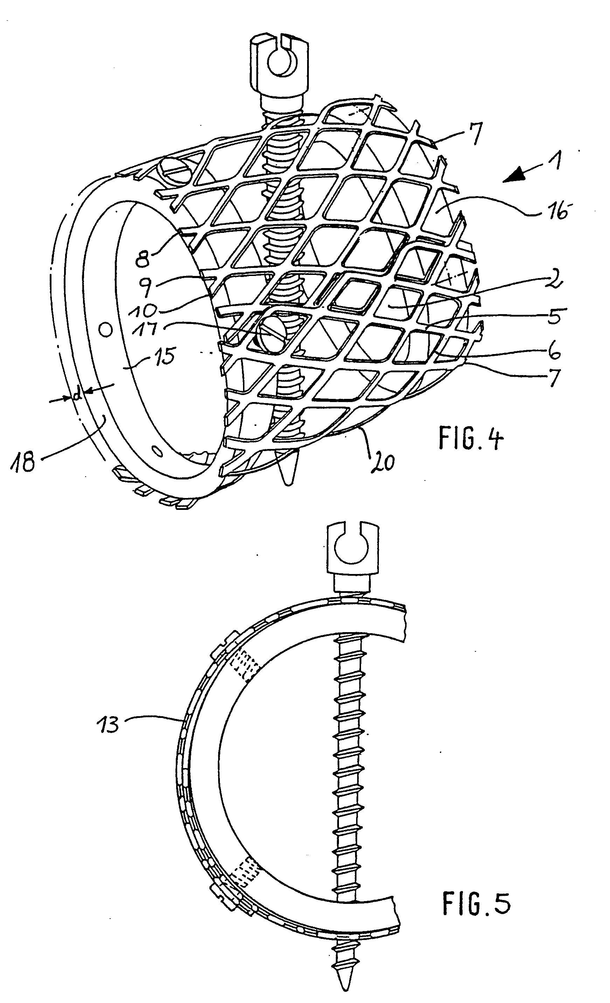

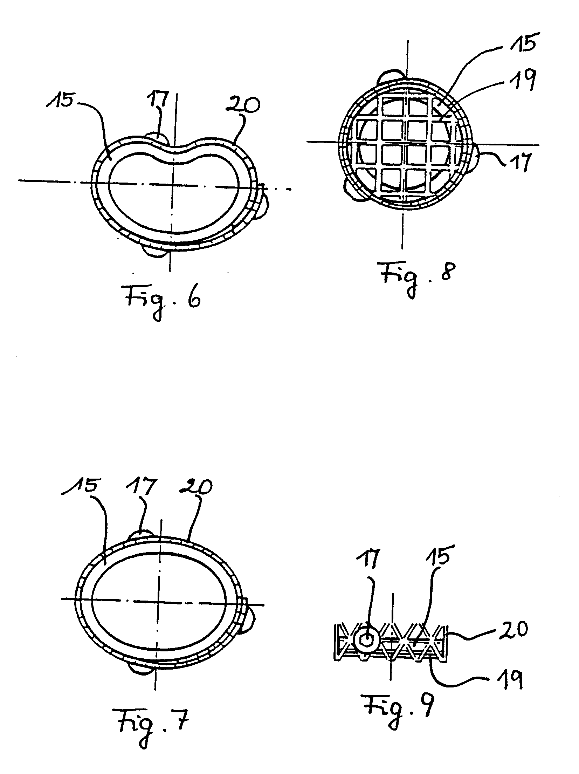

Bei den in den Figuren 4 und 5 gezeigten abgewandelten Ausführungsformen sind einander entsprechende Teile jeweils mit dem gleichen Bezugszeichen gekennzeichnet. Die Ausführungsform unterscheidet sich gegenüber der zuerst beschriebenen dadurch, daß an den beiden gegenüberliegenden Enden des zylindrischen Körpers zylinderförmige Ringe 15, 16 vorgesehen sind, deren Außendurchmesser jeweils mit dem Innendurchmesser des zylindermantelförmigen Elementes übereinstimmt. Der Zylindermantel ist jeweils mittels Schrauben 17 mit dem daran anliegenden Ring 15, 16 fest verbunden. Der jeweilige Ring 15, 16 weist mit seiner außenliegenden Seite 18 einen Abstand d von dem benachbarten Rand 7 bzw. 8 auf. Dieser Abstand liegt vorzugsweise in der Größenordnung von 0,5 bis 2 mm. Beim Einsetzen des Elementes drückt sich dieses bei Belastung mit den über den jeweiligen Ring hervorstehenden Enden in den benachbarten Knochen hinein. Der angrenzende Ring verhindert ein weiteres Eindringen in den benachbarten Knochen. Die gewünschte Größe des Abstandes d wird durch die beabsichtigte Art der Benutzung und die dabei gewünschte Eindringtiefe bestimmt.

Wie aus den Figuren 4 und 5 ersichtlich ist, ist die Abmessung der rautenförmigen Ausnehmungen so gewählt, daß eine Pedikelschraube mit ihrem Gewinde in die Ausnehmung einschraubbar ist, so daß eine zusätzliche einfache Fixierung zwischen dem Platzhalter und den jeweils angrenzenden Wirbeln bzw. Knochenstücken erreichbar ist.

Bei den oben beschriebenen Ausführungsbeispielen ist der Querschnitt jeweils kreisförmig ausgebildet. Bevorzugt wird der Querschnitt jeweils in Abhängigkeit von der Querschnittsform der zu verbindenden Teile verwendet. Für die Verbindung von Wirbeln im lumbalen Bereich weist das Element vorzugsweise einen in Fig. 6 gezeigten nierenförmigen Querschnitt oder einen in Fig. 7 gezeigten ovalen Querschnitt auf.

Die in den Figuren 8 und 9 gezeigte Ausführungsform weist zusätzlich zu den oben beschriebenen Ausführungsformen bodenseitig eine Bodenplatte 19 und auf der gegenüberliegenden Seite eine entsprechende Deckplatte auf. Die Bodenplatte und die Deckplatte sind gitterförmig ausgebildet. Die benachbarten Ringe 15 bzw. 16 dienen jeweils als Widerlager. Diese Ausführungsform ist besonders für die Verbindung poröser Knochen geeignet. Durch die Bodenplatte bzw. Deckplatte wird ein zu starkes Einsinken des Platzhalters in beispielsweise die Wirbeldeckplatte verhindert. Die Bodenplatte bzw. Deckplatte hat vorzugsweise eine gitterförmige Struktur, für die in Fig. 8 ein Beispiel gezeigt ist.

Wie oben ausgeführt, kann das Innere mit Knochenzement ausgefüllt werden. Es ist aber anstelle des Ausfüllens mit Knochenzement auch möglich, im Inneren des Platzhalters eigene oder Fremdknochenstücke einzusetzen, so daß der Platzhalter um das eingesetzte Knochenstück herum die Tragfunktion übernimmt. Sowohl beim Ausfüllen mit Knochenzement als auch beim Einsetzen des eigenen Knochenteiles wird ein besonders schneller Gefäßanschluß mit dem oben beschriebenen Platzhalter erreicht. Außerdem wird ein sicherer Schutz des Rückenmarkkanales gegen Verschleudern des Knochenzementes oder der Knochenstücke erreicht.

## Ansprüche

1. Platzhalter (1), insbesondere für einen Wirbel, dadurch gekennzeichnet, daß ein mantelförmiges Element mit wenigstens einer Ausnehmung (2) in der Wandung vorgesehen ist, dessen oberer und unterer Rand (7, 8) jeweils wenigstens teilweise zackenförmig ausgebildet ist.

2. Platzhalter, insbesondere für einen Wirbel nach Anspruch 1,
dadurch gekennzeichnet, daß ein Teil der Zacken (9-12) zu einer ersten Umfangsrichtung hin und ein Teil zu der zweiten Umfangsrichtung hin geneigt sind.

3. Platzhalter, insbesondere für einen Wirbel nach einem der Ansprüche 1 oder 2,
dadurch gekennzeichnet, daß die Wandung aus einem Blech gebildet ist, welches eine Vielzahl von annähernd viereckigen Ausnehmungen ausweist, die so ausgerichtet sind, daß sich jeweils eine Diagonale im wesentlichen parallel zur Zylinderachse des Elementes erstreckt.

4. Platzhalter, insbesondere für einen Wirbel nach Anspruch 3,
dadurch gekennzeichnet, daß die Ausnehmungen im wesentlichen rautenförmig sind und die Diagonale die Längsdiagonale ist.

5. Platzhalter, insbesondere für einen Wirbel nach Anspruch 3 oder 4,
dadurch gekennzeichnet, daß die Zacken (9-12) jeweils divergierende Abschnitte des Bleches sind.

6. Platzhalter, insbesondere für einen Wirbel nach einem der Ansprüche 1 bis 5,
dadurch gekennzeichnet, daß die Zacken zum besseren Eingreifen in benachbarte Knochenteile angephast sind.

7. Platzhalter, insbesondere für einen Wirbel nach einem der Ansprüche 1 bis 6,
dadurch gekennzeichnet, daß das Element aus einem biokompatiblen bzw. körperverträglichen Material, insbesondre Titan geformt ist.

8. Platzhalter, insbesondere für einen Wirbel nach einem der Ansprüche 1 bis 7,
dadurch gekennzeichnet, daß das Element aus einem doppeltgewickelten Mantel gebildet ist.

9. Platzhalter, insbesondere für einen Wirbel nach einem der Ansprüche 1 bis 8,
dadurch gekennzeichnet, daß das Innere des Mantels mit einem Knochenzement ausgefüllt ist.

10. Platzhalter nach einem der Ansprüche 1 - 9, dadurch gekennzeichnet, daß an wenigstens einem der Enden in einem Abstand (b) vom äußeren Rand (7, 8) ein Anschlag vorgesehen ist, der das Eindringen der Zacken in den benachbarten Knochen begrenzt.

11. Platzhalter nach einem der Ansprüche 1 bis 10,
dadurch gekennzeichnet, daß bodenseitig und/oder deckenseitig eine wenigstens teilweise durchbrochene Bodenplatte zur besseren Abstützung auf dem benachbarten Knochenteil vorgesehen ist.

12. Platzhalter nach Anspruch 1 oder 2,
dadurch gekennzeichnet, daß die Wandung des mantelförmigen Elementes netzförmig ausgebildet ist.

## Claims

1. Gap filler (1), especially for a vertebra, characterized in that a cage-like element with at least one perforation (2) in the wall is provided, and the upper and lower edge (7, 8) of the element is in each case designed to be at least partly serrated.

2. Gap filler, especially for a vertebra according to Claim 1, characterized in that a part of the serrations (9-12) is inclined towards a first peripheral direction and a part is inclined towards the second peripheral direction.

3. Gap filler, especially for a vertebra according to one of Claims 1 or 2, characterized in that the wall is formed of a metal sheet which has a plurality of approximately rectangular perforations which are aligned so that in each case one diagonal extends essentially parallel to the cylindrical axis of the element.

4. Gap filler, especially for a vertebra according to Claim 3, characterized in that the perforations are essentially rhombic, and the diagonal is the longitudinal diagonal.

5. Gap filler, especially for a vertebra according to Claim 3 or 4, characterized in that the serrations (9-12) are in each came diverging sections of the metal sheet.

6. Gap filler, especially for a vertebra according to one of Claims 1 to 5, characterized in that the serrations are chamfered for better engagement in adjacent bone parts.

7. Gap filler, especially for a vertebra according to one of Claims 1 to 6, characterized in that the element is shaped from a material which is biocompatible or tolerated by the body, especially titanium.

8. Gap filler, especially for a vertebra according to one of Claims 1 to 7, characterized in that the element is formed of a doubly wound cage.

9. Gap filler, especially for a vertebra according to one of Claims 1 to 8, characterized in that the interior of the cage is filled with a bone cement.

10. Gap filler according to one of Claims 1-9, characterized in that a stop is provided on at least one of the ends at a distance (b) from the outer edge (7, 8) and limits the penetration of the serrations into the adjacent bone.

11. Gap filler according to one of Claims 1 to 10, characterized in that an at least partially perforated base plate is provided at the bottom and/or top to improve support on the adjacent bone part.

12. Gap filler according to Claim 1 or 2, characterized in that the wall of the cage-like element is designed in the form of a network.

## Revendications

1. Prothèse (1), notamment pour une vertèbre, caractérisée en ce qu'un élément en forme de virole est muni d'au moins un évidement (2) dans sa paroi, les bords supérieur et inférieur (7, 8) présentant au moins partiellement une forme dentelée.

2. Prothèse, notamment pour une vertèbre, selon la revendication 1, caractérisée en ce qu'une partie des dents (9-12) sont inclinées dans un premier sens circonférentiel et une partie dans le deuxième sens circonférentiel.

3. Prothèse, notamment pour une vertèbre, selon l'une des revendications 1 et 2, caractérisée en ce que la paroi est formée d'une tôle qui présente une pluralité d'évidements à peu près quadrangulaires, qui sont orientés de manière qu'une diagonale de chaque évidement s'étende sensiblement parallèlement à l'axe du cylindre de l'élément.

4. Prothèse, notamment pour une vertèbre, selon la revendication 3, caractérisée en ce que les évidements sont sensiblement en forme de losange et que la diagonale est la grande diagonale.

5. Prothèse, notamment pour une vertèbre, selon la revendication 3 ou 4, caractérisée en ce que les dents (9-12) sont des segments divergents de la tôle.

6. Prothèse, notamment pour une vertèbre, selon l'une des revendications 1 à 5, caractérisée en ce que, pour mieux mordre dans les parties osseuses adjacentes, les dents sont biseautées.

7. Prothèse, notamment pour une vertèbre, selon l'une des revendications 1 à 6, caractérisée en ce que l'élément est formé d'une matière biocompatible, ou physiologiquement compatible, notamment de titane.

8. Prothèse, notamment pour une vertèbre, selon l'une des revendications 1 à 7, caractérisée en ce que l'élément est formé d'une virole à double enroulement.

9. Prothèse, notamment pour une vertèbre, selon l'une des revendications 1 à 8, caractérisée en ce que l'intérieur de la virole est rempli d'un ciment osseux.

10. Prothèse selon l'une des revendications 1 à 9, caractérisée en ce qu'au moins à une des extrémités, il est prévu, à une distance (d) du bord extérieur (7, 8), une butée qui limite l'enfoncement des dents dans l'os adjacent.

11. Prothèse selon l'une des revendications 1 à 10, caractérisée en ce que, sur le côté de base et/ou sur le côté du couvercle, est prévue une plaque de base au moins partiellement ajourée, qui sert à donner un meilleur appui contre la partie osseuse adjacente.

12. Prothèse selon l'une des revendications 1 ou 2, caractérisée en ce que la paroi de l'élément en forme de virole est d'une configuration réticulée.

FIG.1

FIG.2

FIG.3

FIG.4

FIG.5

Fig. 6

Fig. 8

Fig. 7

Fig. 9